# EUROPÄISCHE PATENTANMELDUNG

(11) **EP 1 563 853 A1**
(43) Veröffentlichungstag der Anmeldung: **17.08.2005**
(21) Anmeldenummer: 04030399.2
(22) Anmeldetag: 22.12.2004
(51) Int. Cl.: A61L 2/26, A61B 19/02, A61C 19/00

(54) **Siebkorbsystem, insbesondere Sterilisationssiebsystem**

(30) Priorität: 13.02.2004 DE 102004008455
(71) Anmelder: Metallverarbeitung Kögel GmbH, 75038 Oberderdingen (DE)
(72) Erfinder: Kögel, Rolf-Dieter, Dipl.-Kfm., 75038 Oberderdingen (DE)
(74) Vertreter: Bulling, Alexander, Dr.

(57) **Zusammenfassung**

Die Erfindung betrifft ein Siebkorbsystem, umfassend:
- einen Siebkorb (12), insbesondere einen Sterilisationssiebkorb, der einen umlaufenden Rahmen (14) aufweist,
- Einlegelemente (22, 24, 26, 27), insbesondere zur Unterteilung des Korbinnenraums oder zur Anordnung/Halterung von Gut im Korbinnenraum, und
- Befestigungsmittel zur Halterung der Einlegeelemente (22, 24, 26, 27) am Siebkorb (12) oder an anderen Einlegeelementen (22, 24, 26, 27). Die Erfindung kennzeichnet sich dadurch, dass die Befestigungsmittel als Klemmteile (40) ausgebildet sind, die eine erste Klaue (42) zur klemmbaren Halterung am Rahmen (14) des Siebkorbs (12) oder an anderen Einlegeelementen (22, 24) und die eine zweite Klaue (44) zur klemmbaren Halterung eines Einlegeelements (22, 24, 26, 27) umfassen.

Die Erfindung betrifft außerdem ein Klemmteil und/oder ein Einlegeelement für das erfindungsgemäße Siebkorbsystem.

## Beschreibung

Die Erfindung betrifft ein Siebkorbsystem, umfassend einen Siebkorb, insbesondere einen Sterilisationssiebkorb, der einen umlaufenden Rahmen aufweist, umfassend Einlegeelemente, insbesondere zur Unterteilung des Korbinnenraums oder zur Anordnung/Halterung von Gut im Korbinnenraum, und umfassend Befestigungsmittel zur Halterung der Einlegeelemente am Siebkorb oder an anderen Einlegeelementen.

Derartige Siebkorbsysteme, die zusätzlich einen Deckel für den Siebkorb umfassen können, finden beispielsweise in Krankenhäusern, in Kliniken, Labors oder ähnlichen Einrichtungen zur Sterilisation von entsprechenden Gütern Verwendung. Darüber hinaus können die Siebe zur Lagerung, zum sicheren Transport, zur Organisation oder dergleichen von den Gütern eingesetzt werden. Die in einen Siebkorb eingelegten Sterilisationsgüter werden zur Sterilisation samt Siebkorb in einen Sterilisator gegeben.

Um den Siebkorb in unterschiedliche Abteile für beispielsweise Schüttgut zu unterteilen oder um Sterilgut sicher im Siebkorb anzuordnen, finden die Einlegeelemente Verwendung. Zur Halterung der Einlegelemente sind als Befestigungsmittel in Form von Winkelelementen und Schrauben bekannt, mit denen die Einlegeelemente am Siebkorb oder an anderen Einlegeelementen dauerhaft verschraubt und damit gehalten werden. Ein Verschrauben ist vergleichsweise aufwändig. Insbesondere wenn die Lage eines Einlegeelements verändert werden soll oder ein Einlegeelement herausgenommen werden soll, ist ein entsprechend hoher Zeitaufwand zum Lösen der Schrauben erforderlich. Außerdem können Schrauben und/oder Muttern verloren werden; Einlegeelemente können sich dann lösen.

In der WO 98/50083 A1 wird in einem durchbrochenen Träger für die Sterilisation eine Unterteilung durch Einlegeelemente vorgenommen, wobei die zusammensteckbaren Einlegeelemente mit Winkeln verschraubt werden.

Aus der US 6,073,794 A1 ist es bekannt geworden, Einlegeteile zur Einlage in Schubladen mit Klemmteilen untereinander zu verbinden.

Der vorliegenden Erfindung liegt die Aufgabe zugrunde, ein eingangs genanntes Siebkorbsystem bereitzustellen, bei dem das Anordnen und Haltern und Verändern der Lage der Einlegeelemente auf einfache Art und Weise realisiert werden kann.

Diese Aufgabe wird mit einem Siebkorb der eingangs beschriebenen Art dadurch gelöst, dass die Befestigungsmittel als Klemmteile ausgebildet sind, die eine erste Klaue zur klemmbaren Halterung am Rahmen des Siebkorbs oder an anderen Einlegeelementen und die eine zweite Klaue zur klemmbaren Halterung eines Einlegeelements umfassen.

Durch die Ausbildung der Befestigungsmittel als Klemmteile mit jeweils zwei Klauen wird vorteilhafterweise eine einfache und schnelle Anordnung der Klemmteile am Siebkorb und der Einlegeelemente an den Klemmteilen ermöglicht. Schrauben oder Winkel, wie sie bei dem Stand der Technik als Befestigungsmittel vorgesehen sind, können entfallen. Die erfindungsgemäßen Befestigungsmittel sind jeweils einstückig ausgebildet und weisen keine zusätzlichen Bauteile auf. Durch die klemmbare Halterung sind außerdem keine Werkzeuge erforderlich, um die Klemmteile am Siebkorb und/oder an den Einlegeelementen zu haltern. Ferner haben die Klemmteile den Vorteil, dass entlang der Längsrichtung der jeweiligen Klaue ein Verschieben des Klemmteils gegenüber dem Rahmen und/oder des Einlegeelements gegenüber dem Klemmteil möglich ist.

Durch Vorsehen der erfindungsgemäßen Klemmteile kann folglich ein Korbinnenraum flexibel und ohne zusätzliche Hilfsmittel unterteilt werden beziehungsweise können entsprechende Güter ohne zusätzliche Hilfsmittel im Korbinnenraum angeordnet werden.

Bei einer vorteilhaften Ausgestaltung der Erfindung ist vorgesehen, dass die zweite Klaue im Wesentlichen senkrecht zur ersten Klaue angeordnet ist. Hierdurch können die Einlegeelemente senkrecht zu dem Rahmen und damit insbesondere senkrecht zu den Seitenwänden beziehungsweise zum Boden des Siebkorbs angeordnet werden. Je nach Längserstreckung der Klauen kann ein verkippsicheres Anordnen am Rahmen und/oder an anderen Einlegeelementen erzielt werden.

Bei einer bevorzugten Ausführungsform der Erfindung ist vorgesehen, dass die Klauenweite der ersten Klaue im Wesentlichen der Klauenweite der zweiten Klaue entspricht. Dies hat den Vorteil, dass die Klemmelemente flexibel am Rahmen und/oder an den Einlegeelementen angeordnet werden können. Außerdem vereinfacht sich hierdurch die Fertigung der Klemmelemente. Es ist lediglich ein Maß für die Klauenweite beider Klauen einzuhalten.

Ein vorteilhafter Siebkorb ergibt sich dann, wenn der Rahmen umlaufend, insbesondere um den oberen Rand des Siebkorbes verlaufend ausgebildet ist. Dies hat den Vorteil, dass die erste Klaue um den oberen Rahmen beziehungsweise um die obere Kante des Siebkorbes zur Anordnung des Klemmelements greift. Die Klaue kann insbesondere von oben auf den Rahmen aufgesteckt werden.

Vorteilhafterweise ist ferner vorgesehen, dass die Einlegeelemente Halterungsabschnitte aufweisen, deren Breite im Wesentlichen gleich wie die Breite des Rahmens ist. Dadurch wird vorteilhafterweise erreicht, dass zum einen die Einlegeelemente an den Klauen sicher angeordnet werden können. Zum anderen kann die erste Klaue des Klemmteils an den Einlegeelementen, entsprechend der Anordnung an dem Rahmen, angeordnet sein. An der zweiten Klaue kann ein weiteres Einlegeelement gehaltert sein.

Folglich kann gemäß der beschriebenen Ausführungsform der Erfindung das Klemmteil flexibel eingesetzt werden; die erste Klaue kann entweder an dem Rahmen oder an einem anderen Klemmteil angeordnet sein.

Einlegeelemente eines erfindungsgemäßen Siebkorbsystems können eine umlaufend, die Halterungsabschnitte bildende Einfassung aufweisen, die insbesondere ein Siebmaterial einfasst. Solche Einlegeelemente dienen insbesondere zur Unterteilung des Siebkorbinnenraums in mehrere Abteile.

Eine weitere, vorteilhafte Ausführungsform der Erfindung sieht vor, dass der Rahmen und/oder die Halterungsabschnitte beziehungsweise die Einfassung, von Drahtelementen gleicher Stärke gebildet sind. Durch Vorsehen von entsprechenden Drahtelementen am Rahmen und/oder an den Halterungsabschnitten kann auf einfache Art und Weise erreicht werden, dass die Halterungsabschnitte eine gleiche Breite aufweisen wie der Rahmen. Die Klemmelemente können folglich flexibel entweder am Rahmen und/oder an den Halterungsabschnitten angeordnet sein.

Besonders vorteilhaft ist, wenn der Rahmen und/oder die Halterungsabschnitte beziehungsweise die Einfassung, jeweils von zwei parallel zueinander verlaufenden Drahtelementen gleicher Stärke gebildet sind. Die Breite des Rahmens beziehungsweise der Halterungsabschnitte wird folglich durch die Stärke der Drahtelemente vorgegeben. Das Vorsehen zweier, parallel verlaufender Drahtelemente kann außerdem dafür dienen, die freien Enden des Siebkorbmaterials, also eines Sieb- oder Gittermaterials, aufzunehmen. Hierdurch wird gewährleistet, dass Verletzungen an den freien Enden des Siebkorb/Siebgittermaterials sicher ausgeschlossen werden können.

Gemäß einer vorteilhaften Ausgestaltung der Erfindung ist das Klemmteil einstückig aus einem Blechteil gebogen. Das Blechteil kann insbesondere Federblech sein. Derartige Klemmteile sind billig und unaufwändig in der Herstellung. Außerdem weisen sie einen hohe Lebensdauer und gute Materialeigenschaften auf.

Vorteilhafterweise sind die Klauenweiten der beiden Klauen eines Klemmteils gleich oder geringfügig kleiner als die Breite des Rahmens und/oder der Halterungsabschnitte. Hierdurch wird gewährleistet, dass die Klauen den Rahmen beziehungsweise die Halterungsabschnitte sicher umgreifen. Ein selbsttätiges Verschieben des Klemmteils wird insbesondere dann ausgeschlossen, wenn die Klauenbreite geringfügig kleiner als die Breite des Rahmens und/oder den Halterungsabschnitten ist. Die Klemmteile sind dann unter einer Klauenvorspannung am Rahmen und/oder der Halterungsabschnitte gehaltert angeordnet.

Ein vorteilhaftes Klemmteil ergibt sich dann, wenn die Klauen jeweils von zwei Seitenwänden und von einem zwischen den Seitenwänden verlaufenden Boden gebildet sind, wobei eine Seitenwand der einen Klaue mit dem Boden der anderen Klaue verbunden ist. Ein derartiges Klemmteil kann auf einfache Art und Weise aus lediglich einem zusammenhängenden Blechstück gebildet werden.

Dabei kann vorteilhafterweise vorgesehen sein, dass die Seitenwand der einen Klaue im Bereich einer ihrer dem Boden abgewandten freien Kante über einen Verbindungssteg mit der freien Schmalseitenkante des Bodens der anderen Klaue verbunden ist. Bei dieser Ausgestaltung werden auf das Klemmteil wirkende Kräfte gleichmäßig in den Rahmen beziehungsweise in die Einlegeelemente abgeleitet.

Ferner vorteilhaft ist, wenn der mit der Seitenwand der einen Klaue verbundene Boden der anderen Klaue derart zurückversetzt angeordnet ist, dass er im Bereich oder nahe dem Bereich der Mittellängsebene der einen Klaue verläuft. Durch ein derartiges Zurückversetzen wird erreicht, dass zwischen dem Siebkorb und dem Einlegeelement beziehungsweise zwischen zwei miteinander durch ein Klemmteil gehalterte Einlegeelemente lediglich ein geringer oder idealerweise kein Spalt entsteht. Insbesondere dann, wenn der Rahmen und/oder die Halterungsabschnitte über das an sie angrenzende Siebmaterial überstehen, kann dieser Überstand durch das Zurückversetzen des Bodens der anderen Klaue ausgeglichen werden.

Um ein leichteres Einführen der Klaue an dem Rahmen und/oder den Halterungsabschnitten zu ermöglichen, kann erfindungsgemäß vorgesehen sein, dass wenigstens eine Seitenwand wenigstens einer Klaue im Bereich ihrer freien Kante sich nach oben öffnende Einführungsschrägen aufweist. Dadurch kann ein Aufclipsen der jeweiligen Klaue beziehungsweise des Klemmteils auf den Rahmen und/oder die Halterungsabschnitte vereinfacht werden.

Vorteilhafterweise ist ebenfalls erfindungsgemäß denkbar, dass die Seitenwände wenigstens einer Klaue sich nach außen gerichtete Aufweitungen aufweisen. Dadurch wird eine Klaue gebildet, die den Rahmen beziehungsweise die Halterungsabschnitte wenigstens abschnittsweise sicher umgibt. Das Klemmteil kann folglich sicher und dauerhaft an dem Rahmen beziehungsweise den Halterungsabschnitten angeordnet werden.

Ein vorteilhaftes Siebkorbsystem ergibt sich auch dann, wenn die Seitenwände und der Boden des Siebkorbes aus einem tiefgezogenen Drahtgewebe oder Drahtgitter gebildet sind. Die Seitenwände und der Boden des Siebkorbs sind folglich aus dem gleichen Drahtgewebe und/oder Drahtgitter, welches tiefgezogen worden ist. Es ergeben sich insbesondere in den Kantenbereichen keine miteinander zu verbindenden freien Enden des Drahtgewebes oder Drahtgitters. Lediglich die obere, umlaufende Kante des Siebkorbes verbleibt als freie Kante. Vorteilhafterweise kann, wie bereits erwähnt, diese Kante von zwei umlaufenden, parallel zueinander angeordneten Rahmenelementen umgeben sein. Je nach Größe des Siebkorbs können weitere Rahmenelemente, beispielsweise am Boden des Siebkorbs, zur sicheren Auflage, vorgesehen sein.

Die Erfindung betrifft außerdem Klemmteile und/oder Einlegeelemente für erfindungsgemäße Siebkorbsysteme. Insbesondere die Klemmteile können Merkmale aufweisen, die im Vorhergehenden zu dem Siebkorbsystem beschrieben oder erwähnt sind.

Weitere Einzelheiten und vorteilhafte Ausgestaltungen der Erfindung sind der nachfolgenden Beschreibung zu entnehmen, in der die in den Figuren dargestellte Ausführungsform der Erfindung näher beschrieben und erläutert ist. Es zeigen:
- Figur 1: ein erfindungsgemäßes Siebkorbsystem in perspektivischer Ansicht,
- Figur 2: einen Ausschnitt des Siebkorbsystems gemäß Figur 1,
- Figur 3: ein Einlegeelement mit einem an dem Einlegeelement angeordneten erfindungsgemäßen Klemmteil,
- Figur 4: ein an dem Klemmteil gemäß Figur 3 angeordnetes weiteres Einlegeelement,
- Figur 5, 6 und 7: verschiedene Ansichten eines erfindungsgemäßen Klemmteils.

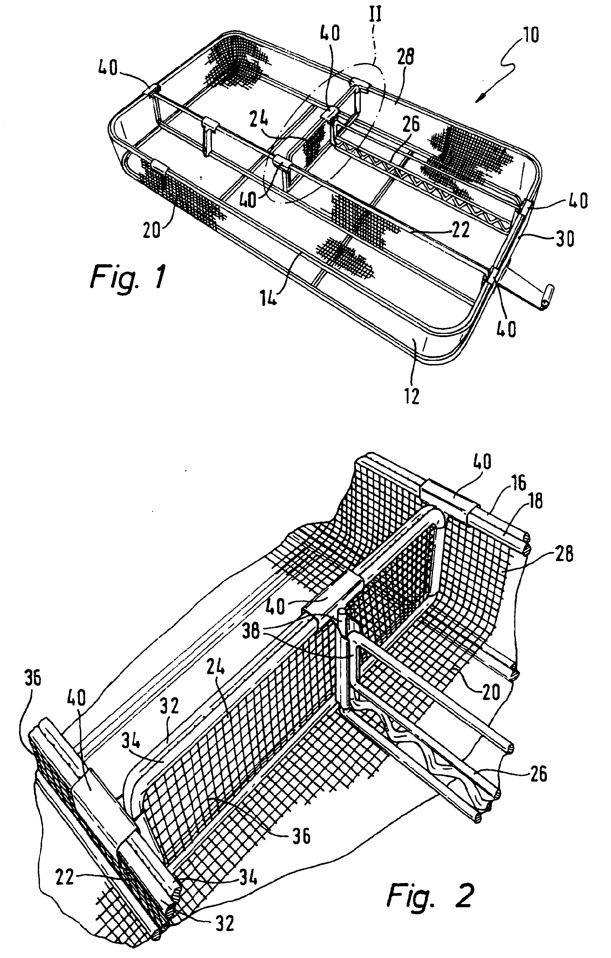

In der Figur 1 ist ein erfindungsgemäßes Siebkorbsystem 10 in perspektivischer Ansicht für Sterilisationsgut gezeigt. Der Siebkorb 12 weist einen an seiner Oberkante angeordneten, umlaufenden Rahmen 14 auf. Der Rahmen 14 wird, wie auch in der Figur 2 zu sehen ist, von zwei parallel, horizontal nebeneinander angeordneten Drahtelementen 16, 18 gebildet. Die beiden Drahtelemente 16, 18 fassen die freie Kante des Siebgewebes 20 ein, aus dem die Seitenwände und der Boden des Siebkorbes 12 gebildet sind. Das Drahtgewebe 20 ist zur Bildung eines Bodens und der Seitenwände tiefgezogen, so dass es lediglich eine freie Kante aufweist, die von den Drahtelementen 16, 18 eingefasst ist.

Wie insbesondere aus der Figur 1 deutlich wird, sind in dem Siebkorb drei Einlegeelemente 22, 24 und 26 angeordnet. Das Einlegeelement 22 erstreckt sich parallel zu den Längswänden des Siebkorbs 12 und unterteilt den Korbinnenraum in insgesamt zwei Teile. Senkrecht zu dem Einlegeelement 22 ist das Einlegeelement 24 angeordnet, das zum einen an dem Einlegeelement 22 und zum anderen an der Längsseite 28 des Siebkorbs 12 befestigt ist. Das Einlegeelement 22 unterteilt den Korbinnenraum folglich in zwei weitere Abteile.

Das Einlegeelement 26 ist zum einen an dem Einlegeelement 24 und zum anderen an der Schmalseite 30 des Siebkorbs 12 angeordnet.

Die beiden Befestigungselemente 22 und 24 weisen jeweils eine umlaufende Einfassung auf, die von zwei parallel zueinander angeordneten Drahtelementen 32, 34 gebildet wird. Die Drahtelemente 32, 34 fassen dabei Siebmaterial in Form von Drahtgewebe ein. Die Drahtelemente 32, 34 weisen eine Stärke auf, die der Stärke der Drahtelemente 16, 18 entspricht.

Das Einlegeelement 26 ist als Halterung für stabförmiges Gut, wie beispielsweise Operationsbesteck, ausgebildet und weist eine entsprechende Form auf. An den dem Einlegeelement 24 und der Schmalseite 30 zugewandten Kanten weist das Einlegeelement 26 zwei parallel zueinander verlaufende Drahtelementabschnitte 38 auf. Die Stärke der Drahtelementabschnitte 38 entspricht der Stärke der Drahtelemente 16, 18 und 32, 34. Zur Halterung der Einlegeelemente 22, 24, 26 sind für jedes Einlegeelement zwei Befestigungsmittel in Form von identisch ausgebildeten Klemmteilen 40 vorgesehen.

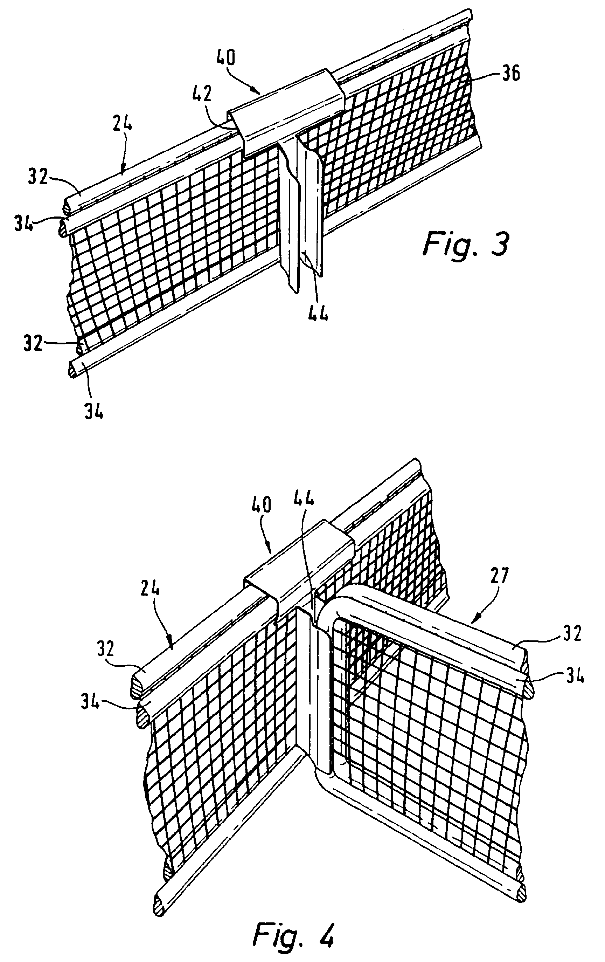

Wie aus den Figuren 3 bis 7 deutlich hervorgeht, weisen die Klemmteile 40 jeweils eine erste Klaue 42 zur klemmbaren Halterung am Rahmen 14 des Siebkorbs oder an anderen Einlegeelementen beziehungsweise deren Halterungsabschnitte auf. Die Klemmteile weisen ferner eine zweite Klaue 44 zur klemmbaren Halterung eines Einlegeelements beziehungsweise dessen Halterungsabschnitt auf. In der Figur 3 ist ein Klemmteil 44 mit seiner ersten Klaue 42 auf zwei parallel zueinander verlaufende Drahtelemente 32, 34 aufgesteckt.

Die Drahtelemente 32, 34 bilden dabei einen Halterungsabschnitt für die Klaue 42 des Klemmteils 40. Die Drahtelemente 32, 34 gehören in Figur 3 zu dem Einlegeelement 24. Anstelle des Einlegeelements 24 kann die Klaue 42 auf den Rahmen 14 des Siebkorbs 12 entsprechendend aufgesteckt sein.

In der Figur 3 ist in der Klaue 44 des Klemmteils 40 kein weiteres Einlegeelement gehaltert.

In der Figur 4 ist in der Klaue 44 des Klemmteils 40 gemäß Figur 3 ein weiteres Einlegeelement 27 gehaltert angeordnet. Das Einlegeelement 27 sieht ebenfalls, wie das Einlegeelement 24, als Halterungsabschnitt zwei umlaufende Drahtelemente 32, 34 vor. Die Klaue 44 nimmt diese beiden Drahtelemente 32, 34 sicher auf.

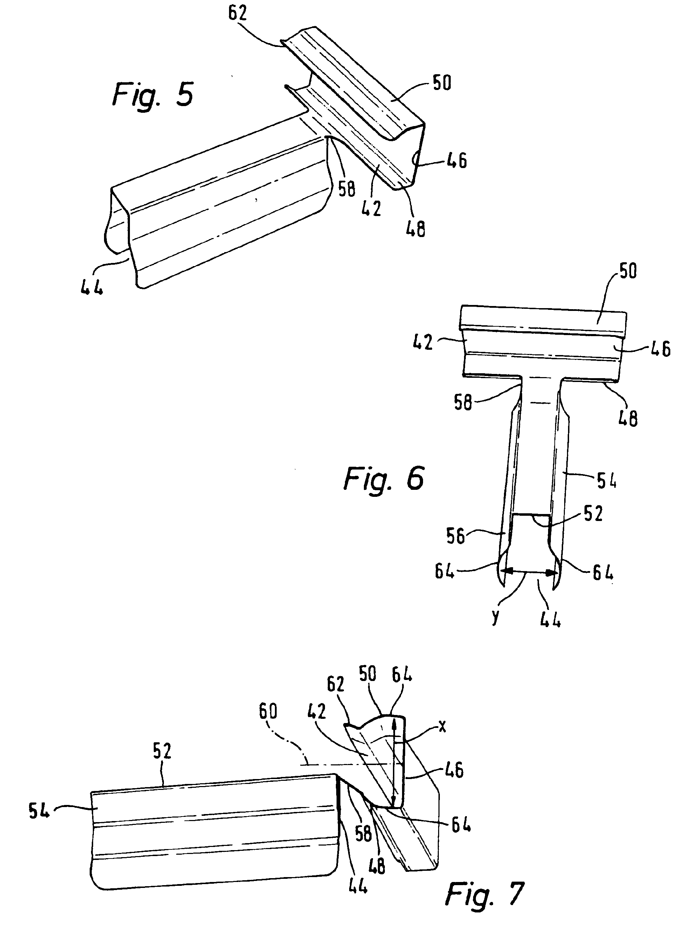

Die beiden Klauen 42, 44 eines Drahtelements 40 verlaufen im Wesentlichen senkrecht zueinander. Dabei entspricht die Klauenweite x der ersten Klaue 42 (vgl. Figur 7) der Klauenweite y der zweiten Klaue 44 (vgl. Figur 6). Beide Klauen 42, 44 sind folglich dazu geeignet, die jeweils parallel zueinander verlaufenden Drahtelemente 32, 34 oder 16, 18 sicher aufzunehmen. Die Klauenweiten x und y sind dabei geringfügig kleiner als die Breite des Rahmens 14 beziehungsweise die Breite der beiden nebeneinander angeordneten Drahtelemente 16, 18 oder 32, 34 samt des dazwischen liegenden Siebmaterials 20, 36. Hierdurch wird gewährleistet, dass das Klemmteil 40 sicher auf den Drahtelementen 16, 18 oder 32, 34 angeordnet werden kann, ohne dass das Klemmteil 40 sich selbsttätig bewegt. Das Klemmteil 44 kann auf die entsprechenden Rahmenelemente 16, 18 beziehungsweise 32, 34 sicher aufgeklemmt werden.

Wie aus den Figuren 5, 6 und 7 deutlich wird, wird die Klaue 42 von einem Boden 46 und zwei Seitenwänden 48, 50 gebildet. Die Klaue 44 wird ebenfalls von einem Boden 52 und zwei Seitenwänden 54, 56 gebildet. Die Seitenwand 48 der ersten Klaue 42 ist in ihrem mittleren Bereich über einen Verbindungssteg 58 mit dem Boden 52 der anderen Klaue 44 verbunden. Das Klemmteil 40 kann insbesondere aus einem Blechteil, beispielsweise einem Federstahlblechteil, gebildet sein.

Wie insbesondere aus der Figur 7 deutlich wird, ist der Boden 52 der Klaue 44 derart zurückversetzt angeordnet, dass er nahe dem Bereich der Mittellängsebene, die durch die strichpunktierte Linie 60 angedeutet ist, der anderen Klaue 42 verläuft. Dadurch wird erreicht, dass bei mit einem Klemmteil 40 zu halternde Teile möglichst nahe aneinander angeordnet werden können. Durch das Rückversetzen des Bodens 52 liegt dieser vorteilhafterweise, wie in Figur 3 und 4 erkennbar ist, sehr nahe an dem Siebmaterial 36 an. Der Boden 52 liegt folglich im Bereich der Ebene des Siebmaterials 36.

Zur sicheren Aufnahme der Drahtelemente 16, 18 beziehungsweise 32, 34 sieht die Seitenwand 50 der Klaue 42 im Bereich ihrer freien Kante eine sich nach außen erstreckende Einführungsschräge 62 vor.

Außerdem sehen die Klauen 42, 44 beziehungsweise deren Seitenwände nach außen gerichtete Aufweitungen 64 zur klauenartigen Aufnahme der entsprechenden Drahtelemente 16, 18 beziehungsweise 32, 34 vor.

Die erfindungsgemäßen Klemmteile 40 haben den Vorteil, dass sie entlang des Rahmens 14 beziehungsweise der Drahtelemente 16, 18 auf den Rahmen 14 beziehungsweise die Drahtelemente 16, 18 aufgeclipst werden können. Aufgrund der entsprechenden Klauenweite der Klaue 42 sind die Klemmteile 40 sicher gehaltert. Mit entsprechendem Kraftaufwand können die Klemmteile 40 händisch und ohne Werkzeug entlang der Längsrichtung ihrer Klaue 42 verschoben werden, an die Stelle, an der ein entsprechendes Einlegeelement in den Siebkorbinnenraum eingelegt werden soll.

Ein entsprechendes Einlegeelement 22, 24, 26, 27 kann dann von oben, entlang der Längsrichtung der Klaue 44, in die jeweilige Klaue 44 eingeschoben werden. Die Einlegeelemente 22, 24, 26 und 27 werden dann an ihren jeweiligen Schmalseiten von den Klemmteilen 40 sicher gehaltert. Die Klemmteile 40 können ohne zusätzliches Werkzeug oder ohne zusätzliche Bauteile an dem Siebkorb 12 beziehungsweise dessen Rahmen 14 und/oder an entsprechenden Einlegeelementen angeordnet werden. Außerdem können die Einlegeelemente in einem nächsten Montageschritt ohne zusätzliches Werkzeug oder ohne zusätzliche Bauteile insbesondere von oben, also senkrecht zur Ebene des Bodens des Siebkorbs, in die Klauen 44 der Klemmteile 40 eingeschoben werden. Ein Unterteilen des Siebkorbinnenraums beziehungsweise ein Anordnen von Einlegeelementen kann damit auf einfache Art und Weise erfolgen. Der Siebkorb 12 wird dabei so unterteilt, dass zu sterilisierendes Gut in den einzelnen Abteilungen des Siebkorbinnenraums vorteilhaft untergebracht und gegebenenfalls gehaltert werden kann.

Sämtliche in der Beschreibung, den nachfolgenden Ansprüchen dargestellte Merkmale können sowohl einzeln als auch in beliebiger Kombination miteinander erfindungswesentlich sein.

## Patentansprüche

1. Siebkorbsystem (10), umfassend
- einen Siebkorb (12), insbesondere einen Sterilisationssiebkorb, der einen umlaufenden Rahmen (14) aufweist,
- Einlegelemente (22, 24, 26, 27), insbesondere zur Unterteilung des Korbinnenraums oder zur Anordnung/Halterung von Gut im Korbinnenraum, und
- Befestigungsmittel zur Halterung der Einlegeelemente (22, 24, 26, 27) am Siebkorb (12) oder an anderen Einlegeelementen (22, 24, 26, 27)
**dadurch gekennzeichnet,**
**dass** die Befestigungsmittel als Klemmteile (40) ausgebildet sind, die eine erste Klaue (42) zur klemmbaren Halterung am Rahmen (14) des Siebkorbs (12) oder an anderen Einlegeelementen (22, 24) und die eine zweite Klaue (44) zur klemmbaren Halterung eines Einlegeelements (22, 24, 26, 27) umfassen.

2. Siebkorbsystem (10) nach Anspruch 1, **dadurch gekennzeichnet, dass** sich die zweite Klaue (44) im Wesentlichen senkrecht zur erste Klaue (42) erstreckt.

3. Siebkorbsystem (10) nach Anspruch 1 oder 2, **dadurch gekennzeichnet, dass** die Klauenweite (x) der ersten Klaue (42) im Wesentlichen der Klauenweite (y) der zweiten Klaue (44) entspricht.

4. Siebkorbsystem (10) nach Anspruch 1, 2 oder 3, **dadurch gekennzeichnet, dass** der Rahmen (14) umlaufend, insbesondere um den oberen Rand des Siebkorbes verlaufend, ausgebildet ist.

5. Siebkorbsystem (10) nach einem der vorhergehenden Ansprüche, **dadurch gekennzeichnet, dass** die Einlegelemente (22, 24, 26, 27) Halterungsabschnitte aufweisen, deren Breite im Wesentlichen gleich wie die Breite des Rahmens (14) ist.

6. Siebkorbsystem (10) nach einem der vorhergehenden Ansprüche, **dadurch gekennzeichnet, dass** die Einlegelemente (22, 24, 27) eine umlaufend, die Halterungsabschnitte bildende Einfassung aufweisen, die ein Siebmaterial (36) einfasst.

7. Siebkorbsystem (10) nach einem der vorhergehenden Ansprüche, **dadurch gekennzeichnet, dass** der Rahmen (14) und/oder die Halterungsabschnitte von Drahtelementen (16, 18, 32, 34) gleicher Stärke gebildet sind.

8. Siebkorbsystem (10) nach einem der vorhergehenden Ansprüche, **dadurch gekennzeichnet, dass** der Rahmen (14) und/oder die Halterungsabschnitte jeweils von zwei parallel zueinander verlaufenden Drahtelementen (16, 18; 32, 34) gleicher Stärke gebildet sind.

9. Siebkorbsystem (10) nach einem der vorhergehenden Ansprüche, **dadurch gekennzeichnet, dass** das Klemmteil (40) einstückig aus einem Blechteil gebogen ist.

10. Siebkorbsystem (10) nach einem der vorhergehenden Ansprüche, **dadurch gekennzeichnet, dass** die Klauenweite (x, y) der beiden Klauen (42, 44) gleich oder geringfügig kleiner als die Breite des Rahmens (19) und/oder der Halterungsabschnitte (32, 34) ist.

11. Siebkorbsystem (10) nach einem der vorhergehenden Ansprüche, **dadurch gekennzeichnet, dass** die Klauen (42, 44) jeweils von zwei Seitenwänden (48, 50; 54, 56) und von einem zwischen den Seitenwänden verlaufenden Boden (46, 52) gebildet sind, wobei eine Seitenwand (48) der einen Klaue (42) mit dem Boden (52) der anderen Klauen (54) verbunden ist.

12. Siebkorbsystem (10) nach Anspruch 11, **dadurch gekennzeichnet, dass** die Seitenwand (48) der einen Klaue im Bereich einer ihrer dem Boden (46) abgewandten freien Kanten über einen Verbindungssteg (58) mit einer freien Schmalseitekante des Bodens (52) der anderen Klaue (44) verbunden ist.

13. Siebkorbsystem (10) nach Anspruch 12, **dadurch gekennzeichnet, dass** der mit der Seitenwand (48) der einen Klaue (42) verbundene Boden (52) der anderen Klaue (44) derart zurückversetzt angeordnet ist, dass er im Bereich oder nahe dem Bereich der Mittellängsebene (60) der einen Klaue (42) verläuft.

14. Siebkorbsystem (10) nach einem der Ansprüche 10 bis 13, **dadurch gekennzeichnet, dass** wenigstens eine Seitenwand wenigstens einer Klaue (50) im Bereich ihrer freien Kanten eine sich nach außen öffnende Einführungsschräge (62) aufweist.

15. Siebkorbsystem (10) nach einem der Ansprüche 10 bis 14, **dadurch gekennzeichnet, dass** die Seitenwände (54, 56) wenigstens einer Klaue sich nach außen gerichtete Aufweitungen (64) aufweisen.

16. Siebkorbsystem (10) nach einem der vorhergehenden Ansprüche, **dadurch gekennzeichnet, dass** der die Seitenwände und der Boden des Siebkorbes (12) aus einem tiefgezogenen Drahtgewebe (20) oder Drahtgitter gebildet sind.

17. Klemmteil (40) und/oder Einlegeelement (22, 24, 26, 27) für ein Siebkorbsystem (10) nach einem der vorhergehenden Ansprüche.
